# EUROPEAN PATENT APPLICATION

(11) **EP 4 498 110 A1**
(43) Date of publication of application: **29.01.2025**
(21) Application number: 23188262.2
(22) Date of filing: 27.07.2023
(51) Int. Cl.: G01R 33/48, G01R 33/56

(54) **SYSTEM AND METHOD FOR ASSESSMENT OF DIFFERENT KINDS OF BODILY TISSUES AND/OR BODILY FLUIDS AND/OR AIR**

(71) Applicant: MRIguidance B.V., 3581 BG Utrecht (NL)
(72) Inventor: Seevinck, Peter Roland, 3572 SH Utrecht (NL); van Stralen, Marijn, 3514 XL Utrecht (NL); Linders, Jara Theodora Wilhelmina, 3543 CE Utrecht (NL)
(74) Representative: DTS Patent- und Rechtsanwälte PartmbB

(57) **Abstract**

The present invention refers to a system for assessment of different kinds of bodily tissues and/or bodily fluids and/or air, in particular air being associated with a human body, wherein, for primary data assessment (320), the processor module (110) is configured to process and/or analyse at least the MRI patient scan data of the first patient such that at least one set of Bone MRI data, in particular synthetic CT data, of the first patient is provided, and/or wherein, for a secondary data assessment (330), the processor module (110) is configured to process and/or analyse at least the MRI patient scan data of the first patient such that at least one set of synthetic T1-weighted image data of the first patient is provided. Furthermore, the present invention refers to a method for training of a processor module (110), a method for providing a primary data assessment (320) and/or a secondary data assessment (330) by such a system (100) and a computer-readable medium.

## Description

The invention refers to a system and a method for assessment of different kinds of bodily tissues and/or bodily fluids and/or air. Moreover, the present invention refers to a method for training as well as a computer-readable medium.

Specific conditions in the human body, e.g. spine conditions, can be related to numerous different causes, including degenerative causes, (congenital) deformities, dislocations, tumors and infections. Even if a patient's anamnesis is key to diagnose these symptoms, medical imaging is frequently performed to confirm the diagnosis. Adequate assessment of underlying soft and osseous tissue defects requires different diagnostic tools.

Magnetic Resonance Imaging (MRI) is a non-invasive and widely used imaging technique offering many image contrasts, which can each highlight different anatomical features, e.g. for detection of several forms of degenerative diseases of e.g. the spine by detection of determining changes in the water content of the biological tissue. These image contrasts are acquired using different sequences, which dictate e.g. when a radiofrequency pulse is sent out and when certain electromagnets in the machine are switched on or off. During most MRI examinations multiple of these image contrasts are acquired, because often no single image contrast is able to clearly show all of the clinically relevant information.

T1-weighted (T1w) and T2-weighted (T2w) MRI scans are two commonly used types of MRI sequences that provide different types of information about the tissues in the body. The T1w sequences represent the T1 relaxation rate at which protons release their absorbed energy - after excitation by the radiofrequency pulse into the transverse plane - into the surrounding tissues and retrieve longitudinal magnetization. Tissues that contain a lot of water, such as cerebrospinal fluid (CSF), appear dark on T1w images, while tissues that contain fatty tissue appear high intense, such as subcutaneous, epidural fat and fat in bone marrow of vertebral bodies. Regions with less water and more fat, such as bone and muscle, appear bright. T1w images are often used to visualize anatomical structures and to detect abnormalities relating to degenerative spine diseases, tumors and early and late subacute hematoma. T1w images are also often used for the detection of spinal stenosis as a good distinction can be made between the fat surrounding the nerve root on T1w images.

Exemplarily in spine imaging, a T1-weighted image is often part of the routine spinal MRI protocol to obtain a clear overview of the anatomy and spine related abnormalities, especially for degenerative disc diseases and metastases. Due to the high contrast of epidural fat with intervertebral discs, cortical bone, nerve roots and thecal sac outline, and fatty degenerations of bone marrow of the discs, T1w scans have an added value for e.g. the evaluation of degenerative spine diseases. Pathologies often identified with T1-weighted scans are then disc herniation, foraminal and spinal stenosis, modic changes, schmorl's nodes and also post-operative fibrosis. In addition, T1w is often used to evaluate bone marrow in the spine, as bone marrow lesions can include fatty tissue.

However, each image contrast requires a certain sequence, with more sequences taking more time and increasing the corresponding costs significantly. A new trend is emerging in MRI in which computer algorithms are used to generate more useful images after scanning to reduce total scan time.

It is an object of the invention to provide a system and a method which allow for an improved and more efficient process and assessment of a first patient's condition, namely for generating appropriate medical imaging data for a first patient, in particular on basis of preferably a set of MRI patient scan data wherein further medical imaging data and/or medical information are generated by a specifically trained processor module of the system. Moreover, it is a further object of the invention to provide a method for training of the system, in particular of the processor module of the system, in order to allow an improved quality and stability, in particular an improved reliability, of the process to generate additional medical imaging data and/or medical information, in particular without any, at least without any substantial, loss of information in comparison to usual medical imaging methods.

According to the present invention, a system is provided for assessment of different kinds of bodily tissues and/or bodily fluids and/or air, in particular air being associated with a human body, is provided which comprises:
- at least one processor module for handling and processing of MRI patient scan data,
- at least one storage module for storing patient data, in particular MRI patient scan data and/or synthetic CT data and/or data associated therewith, of at least one first patient,
wherein at least one data connection is provided between the processor module and the at least one storage module, in particular a bidirectional data connection, such that data are transferrable for processing and/or for storing and wherein the processor module is configured to receive and/or request the at least one set of MRI patient scan data from the storage module or from a MRI scanning device. For primary data assessment, the processor module, preferably comprising a Bone MRI module of the processor module, is configured to process and/or analyse at least the MRI patient scan data of the first patient such that at least one set of Bone MRI data, in particular synthetic CT data, of the first patient is provided, and/or wherein, for a secondary data assessment, the processor module, preferably comprising an image transformation module, is configured to process and/or analyse at least the MRI patient scan data of the first patient such that at least one set of synthetic T1-weighted image data of the first patient is provided.

The basic idea underlying the present invention is to reduce the amount of imaging processes and to replace them by more time and cost-efficient processes, in particular to substitute the amount of imaging sequences for the first patient by generating synthetical images from a minority of first patient's imaging data like e.g. a (single) set of MRI patient scan data of the first patient.

One way to do this is using deep learning, which is a way of training a so-called neural network to learn complex patterns and connections between data. In the context of generating more images, this training is often done by presenting a neural network with at least one input image and at least one corresponding target/output image. The corresponding target image is the image that the neural network is supposed to reconstruct using the input image.

By showing a neural network many of these paired examples, the network is able to learn the underlying connection between the image pairs. After training, one would only acquire the input image, i.e. at least one set of MRI patient scan data of the first patient, and use the neural network to generate the target image from it, which thus is no longer to be acquired by using the MRI machine.

The main motivation is that the computing time needed for the processor module, e.g. comprising a neural network, to generate the corresponding target images is much cheaper than the scan time required to acquire the images using a MRI machine or the like. Moreover, as fewer images need to be produced by means of a MRI machine, more patients can be scanned in the same timeframe.

This idea is taken a step further in Bone MRI, which particularly can be provided as a deep learning-based method that generates synthetic Computed Tomography (CT) images from Magnetic Resonance (MR) images - specifically, Gradient Echo (GRE) images, a type of MR image - using e.g. a neural network that was trained on real MR and CT image pairs.

The contrast of the GRE images acquired for Bone MRI, using the short TE and TR, is strongly influenced by the physical property of the tissues called the T1 relaxation time, making the GRE sequence a T1-weighted sequence. Multiple T1-weighted sequences exist, but the preferred T1-weighted sequence in the clinic is a Turbo Spin Echo (TSE), because it gives better contrast between soft tissues than e.g. a GRE sequence. The TSE is therefore normally performed along with the Bone MRI GRE in a standard examination of e.g. the neck along with other sequences.

The input of the Bone MRI module/application is preferably a high-resolution 3D T1w GRE MRI sequence. The additional scan time of this sequence is limiting the Bone MRI adoption in the regular clinical workflow. Therefore, there is a need for scan time reduction of the Bone MRI solution.

However, according to the present invention this can be resolved by extending the Bone MRI principle with an additional synthetic T1w algorithm. In particular, there is a possibility of generating TSE images from GRE images using e.g. machine or deep learning to replace the real TSE sequence and thereby reduce the total scan time of an examination for a first patient.

As the routine MRI protocol often consists of a combination of scans including 2D clinical T1w TSE images, the overall goal is to generate high-resolution 3D synthetic T1w images. These synthetic T1w images have an increased T1w contrast compared to the high-resolution 3D T1w GRE images used for Bone MRI. This makes the synthetic T1w (sT1w) images more similar to the clinical T1w TSE images and therefore better interpretable to medical end users that are used to this specific type of scan.

The high-resolution 3D T1w GRE is acquired for Bone MRI and is also the scan on which the proposed synthetic T1w solution is based. The 3D T1w GRE is, however, not only useful as input for these applications, but also a widely accepted clinically versatile T1-weighted contrast providing efficient 3D high resolution imaging, allowing multiplanar reformatting in post-processing. Replacing the 2D clinical T1w TSE scan by this sequence adds therefore already extra clinical information to the total patient examination, on top of the Bone MRI and sT1w/synthetic T1-weighted data.

Overall, the present invention, i.e. a combined assessment of Bone MRI/sT1w, allows for a potential application in various different fields with respect to soft tissue structures and segmentation of structures being concerned, like e.g. diagnostic effectivity, surgical planning, (surgery) navigation, robot assisted surgery, AR tooling for intra-surgery feedback and monitoring, 3D printing of anatomies for gaining 3D insight, 3D printing of saw and drill guides, treatment monitoring, mechanical 3D modelling and simulation of forces in spine and soft tissues (for example bone strength, nerves, muscles, blood vessels, intervertebral disc, nucleus pulposus, labrum, cartilage), finite element modeling, mathematical modelling or the like.

In particular, such different applications of the combined Bone MRI/sT1w also originates from having all the generated images in the same geometry, i.e. position, orientation and spacing/resolution, facilitating reading and analysis of the combined set of images resulting from the overall process for which execution the present invention is appropriately configured. For example, a combined visualization/fusion of bone and soft tissue as a subsequent step to imaging bone and soft tissue images (also using segmentations) is provided and eased. Moreover, also benefits like an improved soft tissue contrast in Bone MRI itself after a corresponding (combined) training of the system, i.e. of the processor module, can be achieved.

Preferably all of the data sets, in particular Bone MRI data like synthetic CT data and synthetic T1-weighted image data, are provided in the same, or at least similar, geometry, i.e. position, orientation and spacing/resolution, in order to facilitate the reading of a combined set of images.

Furthermore, it is also possible that the processor module is capable of providing further different types of image data as an output, in particular in the course of the primary or secondary data assessment or by an additional third data assessment. For example, it is also possible that the processor module is further configured to process and/or analyse at least the MRI patient scan data of the first patient such that at least one set of segmentation data of the first patient is provided.

Moreover, in the context of the present invention, the term "3D T1w GRE" shall be understood as high-resolution 3D T1-weighted spoiled gradient echo MRI, which is widely accepted clinically versatile T1-weighted contrast and used as input for the Bone MRI/sT1w application according to the present invention.

The term "2D T1w TSE" shall be understood as 2D T1-weighted turbo spin echo which is a widely used clinical scan with high in-plane resolution, but slices comprising a certain minimum thickness.

The term "Bone MRI data" shall be considered to be generated by the Bone MRI module, on basis of MRI patient scan data of e.g. a first patient, preferably in form of synthetic CT data.

By "synthetic CT data", a data set shall be understood which is generated on basis of at least one set of MRI patient scan data, e.g. of a first patient, by the Bone MRI module in order to replace and/or substitute a separate and real CT imaging sequence.

By the term "synthetic T1w data", in particular a synthetic image with high-resolution 3D T1-weighted properties, it shall be understood in the context of the present invention that on basis of at least one set of MRI patient scan data, e.g. of a first patient, a data set with alternative image properties, e.g. contrast or intensity values, is provided in order to outline and visualize alternative types of information about bodily tissues and/or bodily fluids and/or air.

According to one preferred embodiment the processor module, preferably the image transformation module, is configured to select at least one image property parameter of the synthetic T1-weighted image data, in particular an image contrast, image intensity and/or the like, to which at least one image property value is assignable to and/or determinable for at least one medium, in particular a bodily tissue and/or a bodily fluid and/or air, preferably air being associated with the first patient, by the processor module, in particular by the image transformation module.

In particular, a specific image property parameter can be assigned with a specific image property value in order to establish/provide a preferred configuration of the resulting synthetic T1-weighted image data for the first patient such that specific information are provided for the purpose of assessment of different kinds of bodily tissues and/or bodily fluids and/or air, in particular air being associated with a human body.

According to another preferred embodiment, for the primary data assessment the processor module, in particular the Bone MRI module, is configured to apply a first transfer element on at least the MRI patient scan data, CT patient scan data, Bone MRI storage data, in particular synthetic CT storage data, synthetic T1-weighted image storage data and/or segmentation data of the first patient to provide at least one set of Bone MRI data, in particular at least one set of synthetic CT data, and/or wherein for the secondary data assessment the processor module, in particular the image transformation module, is configured to apply a fourth transfer element on at least the MRI patient scan data, CT patient scan data, Bone MRI storage data, in particular synthetic CT storage data, synthetic T1-weighted image storage data and/or segmentation data of the first patient to provide at least one set of synthetic T1 -weighted image data. The Bone MRI data and the synthetic T1-weighted image data can comprise different T1-weight factors/values.

In particular, in the context of the present invention storage data, like Bone MRI storage data and/or synthetic T1-weighted image storage data, may refer to previous data sets as provided/generated for the first patient at an earlier point in time.

By providing different T1-weight factors for the Bone MRI data and the synthetic T1-weighted image data, different information can be visualized and assessed on basis of the two resulting synthetic data sets.

Moreover, the system according to the present invention is capable of utilizing different types of image data as input for the processor module in order to execute primary and/or secondary data assessment.

Preferably, the system can also be configured to provide such different types of image data as an output, at least on basis of the at least one set of MRI patient image data of the first patient. In particular, the processor module can also be configured to provide e.g. segmentation data of the first patient as an output of a data assessment, namely of the primary/secondary or an additional tertiary data assessment.

Hence, by allowing for different information about the tissue or fluids to be observable additional real medical imaging sequences can be reduced or even completely saved.

Pursuant to another embodiment of the present invention, the processor module, in particular the Bone MRI module, for primary data assessment is further configured:
- to apply a second transfer element on the at least one set of Bone MRI data, in particular synthetic CT data, and/or MRI patient scan data of the first patient such that reduced patient data of the first patient are generated which provide tissue volume information, in particular water and/or fat volume related information and/or the like, of the first patient, and/or
   receiving tissue volume information, in particular water and/or fat volume related information and/or the like, from the MRI scanning device,
- to apply a third transfer element on the reduced patient data and/or the MRI patient scan data of the first patient such that total bone volume information are achieved,
wherein the processor module, in particular the Bone MRI module, is further configured to process and/or analyse the MRI patient scan data, the at least one set of synthetic CT data and/or the reduced patient data, preferably in combination with each other, such that at least one bone volume parameter, in particular bone mineral density, fat percentage, calcium concentration, total density trabecular space and/or the like, of the first patient is determined.

In particular, by transformation and further evaluation of MRI patient scan data additional information can be gathered from the respective data set in order to allow for a detailed analysis of the bone composition, in particular of bone volume parameters like bone mineral density, calcium concentration or further parameters concerning the bone composition.

Moreover, as bone mineral density correlates with bone strength, and thus e.g. with pull-out strength of pedicle screws, the further idea underlying the present invention is to optimize healthcare treatment, namely e.g. for optimized placement of screws in the context of unstable spinal fractures, existing spinal instability, degenerative scoliosis, spinal fusion in anterior strut grafting or the like (see Eur Radiol. 2015 Jun;25(6):1714-20. doi: 10.1007/s00330-014-3529-7. Epub 2014 Dec 7. Quantitative dual-energy CT for phantomless evaluation of cancellous bone mineral density of the vertebral pedicle: correlation with pedicle screw pull-out strength. Wichmann JL 1, Booz C, Wesarg S, Bauer RVV, Kerl JM, Fischer S, Lehnert T, Vogl TJ, Khan MF, Kafchitsas K*.*).

However, as original MRI imaging data do not provide such information in an obvious and immediate manner, the data, as gathered during MRI imaging or by generating synthetic CT data, have to be further processed and/or analyzed in order to emphasize various aspects being specific for corresponding bone compositions such that, in particular by combination of such further processed data, additional information about the bone structure of the first patient and its composition can be gained.

In particular, multiple transfer elements can be provided according to the present invention in order to achieve appropriate data processing starting from MRI patient scan data, synthetic CT data and/or the like for gathering sufficient information for assessment of e.g. bone structures, namely to assess bone volume parameters like mineral density as well as further associated parameters of the bone structures of the first patient.

MRI patient scan data particularly refer to the data of the first patient as acquired during a particular MRI scan. Hence, the MRI patient scan data can refer to specific parts of the first patient's body being focused on during MRI scanning process.

Alternatively, it is also possible that synthetic CT data for specific parts of the first patient's body are already provided.

In the context of the present invention, the processor module can be considered as e.g. a single processor unit, a computer or the like, which is suitable for (appropriate) data processing and/or handling, particularity comprising the Bone MRI module and the image transformation module.

Moreover, the storage module can be any kind of storing device like e.g. a solid state disc, a hard drive or a database being capable of storing and reading/forwarding data, thus for reading and writing of data.

Further, the processor module is configured to apply multiple different transfer elements for the purpose of appropriate data processing, in particular for appropriate processing of MRI patient scan data, in order to provide information about different kinds of tissue like bone volume parameters, e.g. bone mineral density.

In particular, the different transfer elements can be provided as data processing elements and/or algorithms in order to suitably handle, analyse and/or evaluate the individual first patient's data, starting from a MRI patient scan data set of such individual first patient.

Thereby, synthetic CT data can be generated, reduced patient data providing specific information about tissue volume, like e.g. water and/or fat volume related information, of the first patient as well as total bone volume information, preferably separated (or separatable) from the fat volume information of the reduced patient data.

For example, in the context of the present invention it is possible that one MRI patient data set forms the basis of the overall process. These MRI patient data set can preferably be acquired by a single MRI sequence. From such MRI sequence(s), at least one 3D image data set can be acquired which can subsequently be utilized for generation of at least one synthetic CT data set by the Bone MRI module. Moreover, it is also possible that multiple different synthetic CT data sets are generated on basis of the MRI imaging sequence of the individual patient.

In particular, the application of the multiple transfer elements can be considered as a step-by-step data processing for which the processor module of the system is suitably/appropriately configured.

In consequence, the present invention provides a reliable and more efficient process for determination of a.o. bone volume information, i.e. a bone volume assessment, on sole basis of MRI patient scan data.

In the context of the present invention the different transfer elements can be provided as data processing elements, algorithms or the like in order to suitably handle, analyse and/or evaluate the respective sets of data, e.g. starting from at least one set of MRI patient scan data of such individual first patient.

According to another preferred embodiment the processor module, preferably with the Bone MRI module and the image transformation module, is configured to execute primary and secondary data assessment simultaneously or subsequent to each other.

Thus, the data processing can either be executed by the processor module in a stepwise manner or in parallel.

In the context of the present invention, the processor module can be provided in form of a single element or as multiple elements, e.g. comprising a separate Bone MRI module and a separate image transformation module. In particular, the processor module can also be embodied as separate modules being grouped together.

In case the processor module is provided in form of multiple modules, such multiple modules can also make use of at least partially shared networks, weights, etc. to calculate the output images.

In another embodiment the system further comprises a visualization device, in particular a two- or three-dimensional display means, being configured to provide a human perceptible illustration for a first user of the system of
- at least one set of Bone MRI data, in particular synthetic CT data, of the first patient as provided by the processor module, in particular by the Bone MRI module, and/or
- at least one set of synthetic T1-weighted image data as provided by the processor module, in particular by the image transformation module, preferably comprising at least one image property value being assigned to and/or determined for at least one image property parameter for at least one medium, in particular a bodily tissue and/or a bodily fluid and/or air, in particular air being associated to a human body.

In particular, the visualization device may be configured to allow for combined visualization/fusion of bone and soft tissue as a subsequent step to imaging bone and soft tissue images, preferably also being capable of using/visualizing further image data like e.g. segmentations.

In the context of the present invention, image property parameter may be any kind of image contrast, image intensity, pixel spacing/resolution or the like, to which one or multiple image property values can be assigned in order to specify the corresponding image property setting(s).

According to another object of the present invention, a method for training of a processor module is provided, preferably of a Bone MRI module and/or an image transformation module, in particular of a system according to one of the preceding claims, wherein the method comprises the steps of:
- requesting and/or receiving MRI patient scan data, CT patient scan data, Bone MRI data, in particular synthetic CT data, synthetic T1-weighted image data and/or segmentation data of a plurality of patients as at least one set of training data comprising training input data and training output data,
- applying the at least one set of training data to the processor module, preferably to the Bone MRI module and/or the image transformation module, for training of the Bone MRI module, in particular the first transfer element, and/or of the image transformation module, in particular the fourth transfer element, to be configured to generate
- at least one set of Bone MRI data, in particular synthetic CT data, by the processor module, preferably by the Bone MRI module, and/or
- at least one set of synthetic T1-weighted data by the processor module, preferably by the image transformation module,
wherein training of the processor module is preferably achieved by optimizing at least one free transfer parameter of the Bone MRI module, in particular the first transfer element, and/or at least one free transfer parameter of the image transformation module, in particular of the fourth transfer element, and wherein training output data are preferably Bone MRI data and/or synthetic T1-weighted image data of the plurality of patients.

In particular, the method for training according to the present invention can be provided and executed as a separate training of the processor module, namely of the Bone MRI module and the image transformation module, or as a combined training method.

In more detail, a combined training method may imply that a set of MRI data, e.g. 3D T1w GRE data, of a plurality of patients is provided as input data and 2D T1w TSE as well as CT data of the plurality of patients as a set of corresponding (target/output) data are given such that the processor module, i.e. the Bone MRI module and the image transformation module, can be configured to arrive at and to provide Bone MRI data, i.e. synthetic CT data, and synthetic T1-weighted image data for such plurality of patients. Such Bone MRI data, i.e. synthetic CT data, and synthetic T1-weighted image data may also be part of the storage data of such plurality of patients for the purpose of training and/or for the purpose of comparison of progress of such training.

The different types of training data as provided can be separated into training input data and training output data according to the purpose of the processor module to be fulfilled after its training. For example, the training input data can be considered to solely comprise MRI patient scan data of a plurality of patients while the training output data can be considered to comprise Bone MRI data, i.e. synthetic CT data, synthetic T1-weighted image data and segmentation data of the plurality of patients. In this case, the processor module will be trained to generate Bone MRI data, i.e. synthetic CT data, synthetic T1-weighted image data and segmentation data from at least one set of MRI patient scan data of the first patient as input data for data assessment.

In another example, the training input data can be considered to comprise MRI patient scan data and segmentation data of the first patient while the training output data can be considered to comprise Bone MRI data, i.e. synthetic CT data, synthetic T1-weighted image data. In this case, the processor module will be trained to generate Bone MRI data, i.e. synthetic CT data, and synthetic T1-weighted image data from at least one set of MRI patient scan data and at least one set of segmentation data of the first patient as input data for data assessment.

Hence, the whole process can be further extended to also generate segmentations as output to be provided by the appropriately configured processor module.

These segmentations can be overlayed on both the Bone MRI data, i.e. synthetic CT data, and synthetic T1-weighted image data, as they are also generated based on the same input. This can be segmentations of bone structures, but also of soft tissue structures such as the spinal canal, nerves or blood vessels.

Providing such segmentations can also cause benefit for the visualization of fused/combined Bone MRI data and synthetic T1-weighted images wherein both, bone and soft tissue, can be visualized in one common image.

Moreover, combined training of the processor module, namely of the Bone MRI module and the image transformation module, can provide benefits to the synthetic CT data (i.e. Bone MRI data) with more information on image intensities like soft tissue contrast (coming from the 2D T1w TSE as target), which may allow soft tissue assessment on Bone MRI data alone, preferably using a dedicated soft-tissue viewing window.

Additionally or alternatively, a combined training can provide benefits for the synthetic T1-weighted images with more information on edge sharpness of tissue structures such as in particular bone, and 3D, high resolution properties (coming from the CT as target), that results in synthetic T1-weighted images that are also sharply delineated and of 3D, high-resolution quality.

Furthermore, because CT is a different modality than MRI, the typically known MRI artefacts, such as motion artefacts, bias field, folding or flow artefacts, are not present on CT. Therefore, using the CT as target/output in the combined training can benefit synthetic T1w image data by presenting an artefact-free alternative, resulting in synthetic T1w images that are minimally impacted by artefacts present in the input MRI.

By such training method according to the present invention, the corresponding processor module shall be appropriately configured in order to be capable of providing an improved quality and stability, i.e. an improved reliability, for the generation of synthetic images on basis of at least one set of MRI patient scan data. Therefore, the processor module is preferably trained to cope with various difficulties and challenges.

For example, the processor module may be configured to cope with different image spacings/resolutions, wherein e.g.
- training input data and/or input data in general can comprise 3D high resolution gradient echo MRI with (thin/thinner) two-/three-dimensional volumes, like e.g. slices or patches, while
- training output data can comprise 2D T1-weighted TSE data with (thick/thicker) two-/three-dimensional volumes, like e.g. slices or patches, and
- output data to be generated can comprise 3D T1-weighted image data with (thin/thinner) two-/three-dimensional volumes, like e.g. slices or patches (having same resolution as the input data).

In particular, there is a challenge in dealing with high-resolution input, providing high-resolution output, that in the training process should be compared to a low-resolution target/output.

However, such challenges can be resolved by the approach of the present invention, preferably by the use of resampling operations and/or geometry meta-data, in order to minimize the information loss and interpolation of information during training of the processor module.

In another embodiment of the method according to the present invention, on basis of the at least one set of training data of the plurality of patients, the processor module, preferably the image transformation module of the processor module, in particular the fourth transfer element, is trained to be configured to assign and/or determine at least one image property value to at least one image property parameter, in particular an image contrast, image intensity and/or the like, for at least one medium, in particular a bodily tissue and/or a bodily fluid and/or air, of the synthetic T1-weighted image data, and/or wherein the processor module, preferably the image transformation module, in particular the fourth transfer element, comprises a machine learning element, a deep learning element, a neural network element and/or the like.

In particular, the processor module, preferably the image transformation module, can be provided with or make use of convolutional neural network (CNN) based architectures such as HighRestNet or U-Net, attention based architectures such as transformer networks or the like.

A CNN is a type of artificial neural network that is specifically designed for processing and analyzing data with a grid-like structure, such as images (e.g. 3D medical images). CNNs have been widely used in computer vision tasks also relevant to the field of medical imaging, including image classification, image segmentation and image synthesis. By repeating the training process on a large dataset, CNNs can learn to automatically extract hierarchical representations of data, enabling them to recognize complex patterns and make accurate predictions on new, unseen inputs. On the other hand, attention based networks enable the model to focus on different parts of the input sequence when making predictions for a particular output position. By incorporating attention mechanisms into deep learning models, the models gain the ability to selectively attend to different parts of the input sequence, effectively capturing relevant information and improving performance in sequence-to-sequence tasks. In particular transformers rely on an attention mechanism to capture dependencies between different parts of an input sequence. This input sequence can for example be a combination of image volumes and other meta-data that describe relevant properties of the data. By leveraging self-attention mechanisms and parallel processing, transformer networks are able to capture long-range dependencies in sequences more effectively compared to traditional architectures, such as a CNN.

Pursuant to another preferred embodiment of the present invention, applying the at least one set of training data for training of the image transformation module, in particular of the fourth transfer element, further comprises the following steps:
- selecting body region data, in particular at least one two- or three-dimensional volume, from the at least one set of training data, in particular from the training input data, with the body region data comprising and/or being separated/separatable into a set of multiple sub-volume data, preferably a stack of multiple sub-volume data, in particular multiple two- or three-dimensional sub-volumes,
- applying the selected body region data, in particular the multiple sub-volume data, to the processor module such that the processor module, preferably the image transformation module, in particular the fourth transfer element, transfers the corresponding training input data to the corresponding training output data in order to be configured to generate at least one set of synthetic T1-weighted image data.

In particular, the step of selecting body region data may also comprise intermediate steps of registration and/or matching of different data sets in order to allow an appropriate overlay/fitting of different data sets.

Moreover, two or three dimensional sub-volumes can be provided in form of two or three-dimensional slices, three-dimensional patches or the like.

In a further preferred embodiment, applying the at least one set of training data, in particular the selected body region data, for training of the image transformation module further comprises the following steps:
- selecting at least one sub-volume data from the training input data and at least one, preferably one, corresponding sub-volume data from the training output data,
- providing and applying at least one resampling operation parameter as the at least one free transfer parameter of the fourth transfer element to the at least one selected sub-volume data in order to modify the geometry of the selected sub-volume data, in particular to modify at least one position parameter, an orientation parameter, a spacing parameter or the like of the selected sub-volume data, by assigning a resampling operation value to the resampling operation parameter
such that the processor module , in particular the image transformation module, is configured to transfer a geometry of training input data, in particular of the selected sub-volume data, to the corresponding training output data, in particular to the corresponding sub-volume data of the training output data.

In particular, for different sub-volumes of the selected body region data, different resampling operation values can be applied. For example, with regard to a separation into five sub-volumes, a statistical distribution of resampling factor values can be applied, e.g. to provide multiple resampled sub-volumes ranging from lower to higher resolutions than the resolution of the original sub-volume. Important is that there is a good balance between the resulting high-resolution and low-resolution sub-volumes, in order to expose the module to a wide variation of spacings/resolutions during training, making the module spacing/resolution invariant, meaning not biased to a particular resolution.

The resampling operation parameter can be provided in form of a single variable/parameter, a vector, a matrix or the like. Moreover, the resampling operation parameter may be considered as a weighing parameter/weight factor which may allow for emphasizing particular sub-volumes out of a stack of sub-volumes when being applied to such (selected) body region area.

Further, when the network would not be able to deal with e.g. multiple different spacings/resolutions, and would thus not be spacing/resolution invariant, it cannot use all information of the high-resolution input and corresponding output, when compared to the low-resolution output, which results in a loss of information during training. A simple solution would be to up-sample the low-resolution output, but that would result in the network optimising its output against interpolated information, which might result in an unrealistic representation of real-life practice.

Therefore, the appropriate configuration and training of the processor module is directed to the overall aim to minimize the loss of information and interpolation when the data transformation, e.g. from higher pixel resolution to lower pixel resolutions or the other way around, takes place such that the quality and reliability of the resulting synthetic image data, e.g. of the synthetic T1-weighted image data, is improved and maximized, in particular in comparison to image data being providable by a separate, additional medical imaging sequence.

According to another embodiment, applying the at least one set of training data, in particular the selected body region data, for training of the image transformation module further comprises the following steps:
- selecting at least one sub-volume data from the training input data and at least one, preferably one, corresponding sub-volume data from the training output data,
- extracting geometry meta-data from the training input data and applying such geometry meta-data to the processor module, in particular to the image transformation module, such that the processor module, in particular the image transformation module, is configured to modify at least one geometry parameter, in particular at least one position parameter, an orientation parameter, a spacing parameter or the like of the selected sub-volume data, as the at least one free transfer parameter of the fourth transfer element

in order to transfer a geometry of training input data, in particular of the selected sub-volume data, to the corresponding training output data, in particular to the corresponding sub-volume data of the training output data.

Geometry meta-data can be used to provide information to the processor module during training about the geometrical properties, e.g. spacing/resolution properties of the data that the module is processing, in particular the training input data and training output data.

This way the processor module learns to link e.g. this specific spacing/resolution information, namely geometrical information, to specific properties of the sub-volume data/body region area.

For example, a sub-volume is provided together with the geometry meta-data corresponding to this sub-volume (e.g. a spacing/resolution of 1x1x2 mm). The module learns the association between the provided spacing/resolution and the corresponding image properties, which enables the processor module to process volumes of any resolution and to output volumes of any spacing/resolution. This is another way to create a spacing/resolution invariant network.

In a further embodiment applying the at least one set of training data, in particular the selected body region data, for training of the image transformation module further comprises the following steps:
- selecting multiple sub-volume data of the training input data and at least one, preferably one, corresponding sub-volume data from the training output data,
- providing and applying at least one resampling operation parameter as the at least one free transfer parameter of the fourth transfer element to the at least one selected sub-volume data in order to modify the geometry of the selected sub-volume data, in particular to modify at least one position parameter, an orientation parameter, a spacing parameter or the like of the selected sub-volume data, by assigning at least one resampling operation value to the resampling operation parameter
such that the processor module, in particular the image transformation module, is configured to transfer a geometry of training input data, in particular of the selected sub-volume data, to the corresponding training output data, in particular to the corresponding sub-volume data of the training output data. The at least one resampling operation value is randomized, preferably on basis of a uniform random distribution or the like, wherein within the selected body region data different resampling operation values are applicable for and assigned to at least two of the multiple sub-volume data of the training input data, and wherein during subsequent executions of the method different selections, in particular shifted selections within the selected body region data, of the multiple sub-volume data from the training input data are used and the selection of sub-volume data from the training output data maintains the same.

According to present invention, a randomization can also be based on e.g. a gaussian distribution with a maximum resampling operation value being applied for the middle/center sub-volume whereby lower resampling factors may be applied to the sub-volumes at the end sides of the selected body region data.

In this context, the goal may be to find the best match between the high-resolution output of the processor module in comparison to the low-resolution training output data, without the processor module recognizing any patterns in the matching of sub-volumes, which might lead to undesired and biased optimization of the model towards only those sub-volumes, i.e. slices, patches or the like, it is actively matched to.

In particular, by applying such randomization the processor module shall be prevented from adapting to specific patterns in the training data during the learning process.

Thus, an improved quality and robustness/stability of the process to particularly generate synthetic T1-weighted image data can be provided.

According to another embodiment, the step of providing and applying at least one resampling operation parameter as free transfer parameter of the fourth transfer element further comprises the following steps:
- identifying a two- or three-dimensional profile of the at least one sub-volume data of the training input data,
- pre-determining and/or optimizing at least one resampling operation value on basis of the identified profile for the respective sub-volume data,
- applying the at least one pre-determined and/or optimized resampling operation value to at least one of the multiple sub-volume data of the training input data,
such that the processor module, in particular the image transformation module, is configured to transfer a geometry of training input data, in particular of the selected sub-volume data, to the corresponding training output data, in particular to the corresponding sub-volume data of the training output data.

In the context of the present invention, the term "profile" may preferably refer to and describe the actual image voxel intensity values that correspond to a certain geometry. For example, the anatomical area covered by a certain thickness (geometry) of the selected body region or a corresponding sub-volume results in specific image intensities, image contrasts, noise-levels/ratios and/or the like of the respective image data.

Based on such characteristics of the image data, i.e. of the respective body volume and/or the respective sub-volume data, the at least one resampling operation parameter can be configured in the course of the training process.

In particular, the method according to present invention in one preferred embodiment allows for an optimization based on the sub-volume's individual profile.

Hence, by such optimization, the fourth transfer element, namely at least one free transfer parameter of the fourth transfer element being available for adaption of the fourth transfer element in the course of the learning process, can be modified in order to further improve the accuracy, reliability and suitability of sT1w/synthetic T1-weighted image data to be generated on basis of at least one set of MRI patient scan data after the learning process has been executed.

According to another object, the present invention further refers to a method for providing a primary data assessment and/or a secondary data assessment by a system according to the present invention.

In particular, such method for primary/secondar data assessment can make use of the processor module of the system according to present invention being trained in accordance with a method for training according to the present invention.

Moreover, in the light of an additional object of the present invention a computer-readable medium is provided, comprising instructions which cause at least one computer, at least one processor and/or the like to execute the method for training of the processor module, in particular of the Bone MRI module and/or the image transformation module, according to the present invention and/or to execute the method according to the present invention.

Further details and advantages of the present invention shall now be disclosed in the connection with the drawings.

It is shown in:
- **Fig. 1**: a schematic illustration of an embodiment of a system for assessment of different kinds of bodily tissues and/or bodily fluids and/or air;
- **Fig. 2**: a schematic flow-chart illustrating an embodiment of the method for training of a processor module of the system;
- **Fig. 3**: a schematic flow-chart illustrating another embodiment of the method for training of a processor module of the system;
- **Fig. 4**: a schematic flow-chart illustrating an embodiment of a method for providing and executing primary and/or secondary data assessment; and
- **Fig. 5**: a schematic flow-chart illustrating another embodiment of a method for providing and executing primary and/or secondary data assessment.

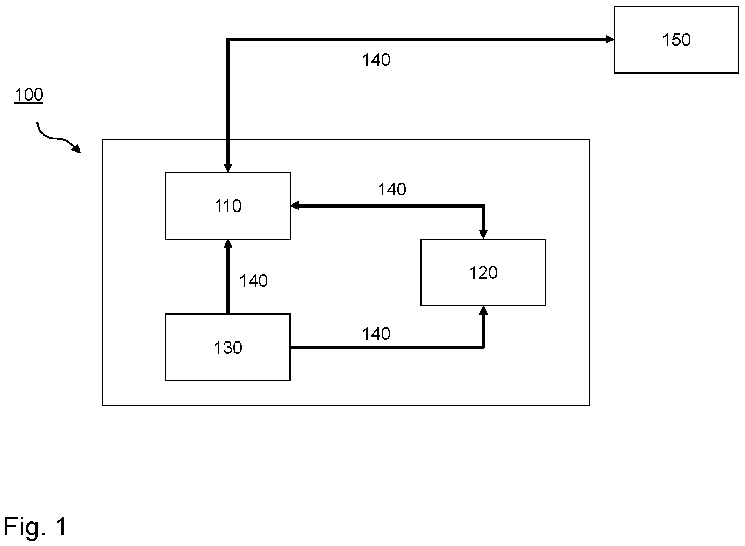

**Fig. 1** shows a schematic illustration of an embodiment of a system 100 for assessment of different kinds of bodily tissues and/or bodily fluids and/or air, in particular air being associated with a human body.

According to Fig. 1 the system 100 comprises at least one processor module 110 and at least one storage 120.

The processor module 110, as schematically illustrated in Fig. 1, can comprise a Bone MRI module and an image transformation module, either in combined form or as separate modules being e.g. arranged together. Alternatively, the Bone MRI module and an image transformation module can be provided completely separate from each other, causing the processor module 110 to be embodied as a multi-part module.

Optionally, the system 100 can further comprise at least one MRI scanning device 130.

The processor module 110 and the storage module 120 can be connected with each other by a bidirectional data connection 140.

Moreover, the optional MRI scanning device 130 can be connected to the processor module 110 and/or the storage module 120.

In particular, the data connection(s) 140 with the MRI scanning device 130 can be provided as unidirectional data connections 140.

Alternatively, the system 100 can be provided without at least one MRI scanning device 130.

In particular, one or multiple MRI scanning devices 130 can for example be situated at remote locations, with the MRI scanning device 130 being in data connection with the system 100, in particular with the at least one processor module 110 and the at least one storage 120 of the system 100.

Moreover, as a further alternative it is also possible that the at least one processor module 110 and the at least one storage module 120 are implemented in the at least one MRI scanning device 130.

With respect to the MRI scanning device 130, the system 100 can be provided as an embedded system, preferably embedded in a corresponding MRI scanning device 130.

Furthermore, a remote data storage and/or a server 150 can be connected with the system 100, as shown in Fig. 1.

In particular, the remote data storage 150 can comprise a data connection 140, preferably a bidirectional data connection 140, with the processor module 110 of the system 100.

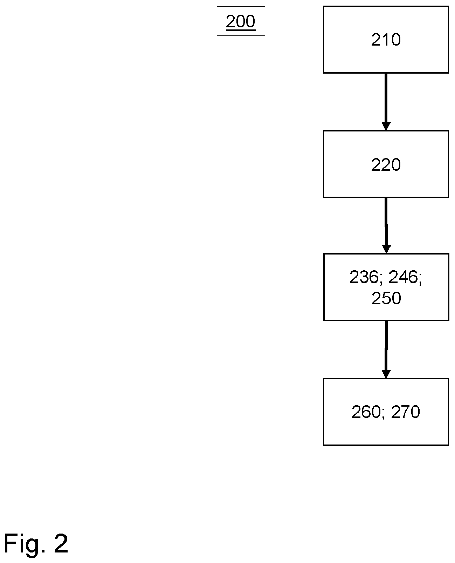

In **Fig. 2** a schematic flow-chart is shown which illustrates an embodiment of the method 200 for training of a processor module 110 of the system 100.

In a first step, at least one set of training data of a plurality of patients is requested and/ or received 210 by the processor module 110.

In particular, the processor module 110 can receive/request such training data from a data storage like a local internal data storage, a (remote) server or the like.

Moreover, the training data of a plurality of patients can comprise various kinds of data, like MRI patient scan data, CT patient scan data and/or Bone MRI data, in particular synthetic CT data, synthetic T1-weighted image data and/or segmentation data of a plurality of patients.

Moreover, the training data can comprise and can be separated into training input data and training output data.

Preferably, corresponding training input data can be MRI patient scan data of the plurality of patients, CT patient scan data, segmentation data and/or the like.

Preferably, corresponding training output data can be synthetic CT data of the plurality of patients, synthetic T1-weighted image data and/or the like.

In a next step, the training data, namely the training input data and the training output data, are applied 220 on the processor module.

Thereby, in a next step the processor module 110, in particular a first, second, third and/or fourth transfer element, is/are trained and configured on basis of transferring 236; 246; 250 the training input data as provided into the training output data as provided.

Thereby, the processor module 110 can be trained and configured to modify and transfer the training input data in order to fit/match/correspond to the training output data. For example, the orientation or position of such training input data can be modified in such process, or e.g. the type of information as provided by the training input data can be modified in order to fit/correspond to the training output data. Hence, it is also possible that, according to the transfer output data, new or different types of image data can be requested to be provided/generated by the processor module 110 on basis of the training input data in the course of the training process.

Moreover, the processor module 110 can be trained, in parallel or in subsequent steps, to determine and/or assign 250 at least one image property value for/to at least one image property parameter, like e.g. an image contrast and/or and image intensity. Namely, the processor module can be trained to adapt such image property value(s) of the training input data to match/corresponding with/to the training output data, in order to ease and improve a clinical assessment of the resulting image data to be generated and provided by the processor module.

The different transfer elements can be provided as and comprise a machine learning element, a deep learning element, a neural network element and/or the like.

The training of the processor module 110 can be considered as a modification and adaption of free transfer parameters of the different transfer elements, namely of the corresponding machine learning/deep learning/neural network elements/models.

In particular, the training of such elements/models can also comprise and refer to a cross-fitting of image information from the different imaging sequences as provided by the available training data, like e.g. a cross-combination and cross-fitting of the high-resolution MRI patient scan data, CT patient scan data, 2D clinical T1-weighted images, high-resolution 3D synthetic T1-weighted image data and/or segmentation data of the plurality of patients.

Finally, the processor module 110, comprising, separately or combined, the Bone MRI module and the image transformation module, is provided 260; 270 in its appropriately configured form, namely being configured to generate Bone MRI data, i.e. synthetic CT data, and sT1w/synthetic T1-weighted image data on basis of at least one set of MRI patient scan data of a first patient.

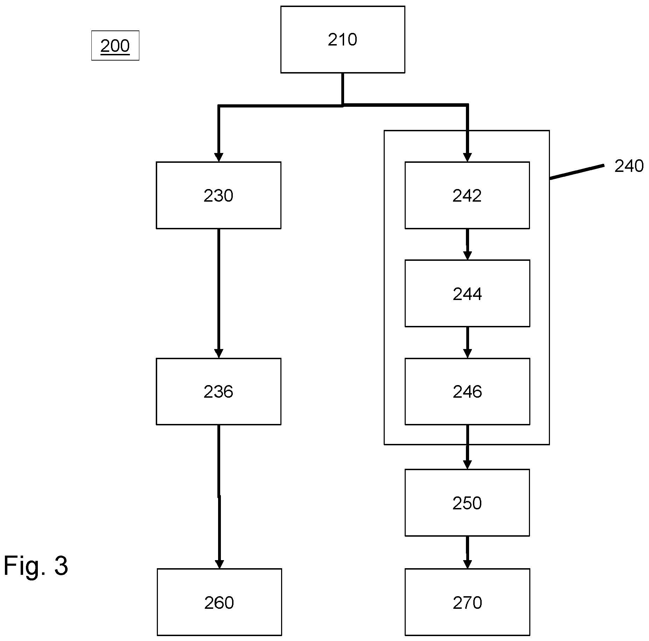

**Fig. 3** illustrates a schematic flow-chart of another embodiment of the method for training of a processor module of the system.

In particular, the scheme for training of the processor module 110 according to Fig. 3 differs from Fig. 2 by the training of the Bone MRI module and the image transformation module being preferably executed separately from each other, namely in two parallel processes being executed simultaneously or sequentially.

Moreover, Fig. 3 illustrates the application 240 of a set of training data on the image transformation module in more detail, which may, however, be executed in the course of the training method 200 according to Fig. 2 in similar manner.

In more detail, for training of the Bone MRI module, the at least one set of training data of a plurality of patients is applied 230 on the Bone MRI module of the processor module 110.

In a next step, the Bone MRI module, in particular the first, second and third transfer element, is/are trained and configured by implementing the necessary transfer of the training input data as provided to the training output data as provided.

Finally, the Bone MRI module is provided in its configured form 260.

Separately, the image transformation module of the processor module 110 can be trained and configured appropriately by applying 240 the given training input data and the given training output data accordingly.

In more detail, for training of the image transformation module, a specific body region area/data set can be selected 242 which may comprise at least one or multiple, in particular a stack of, sub-volume data.

In a next step at least one sub-volume data is selected 244 from such body region area. In particular, a stack/multiple sub-volume data, being preferably arranged next to each other, can be selected from the training data of a plurality of patients.

In a further step, the image transformation module is trained on basis of the transformation 246 of the training input data into the training output data, in particular concerning the respective sub-volume data as selected.

Such training and configuration of the image transformation module can be executed by adapting free transfer parameter of the image transformation module, in particular of the fourth transfer element, in order to allow the transformation module to execute to execute the transfer of training input data into training output data as provided.

Such adaption of the image transformation module, namely of such free transfer parameters, may be provided on basis of one or by combination of multiple different approaches.

Such approaches may, for example, refer to
- assigning specifically appropriate resampling operation values to at least one resampling operation parameter,
- (further) optimization of such resampling operation values, and/or
- extracting and applying geometry meta-data.

Thereby, the transfer of training input data to transfer output data may refer to a modification of a geometry of the respectively selected sub-volume data, for example at least one position parameter, at least one orientation parameter, at least one spacing/resolution parameter and/or the like.

In particular, the image transformation module can be trained to transfer at least one set of high-resolution 3D T1w GRE MRI data, as training input data and as starting point for a regular secondary data assessment 330, into a set of high-resolution synthetic T1-weighted image data, as also given in form of corresponding training output data.

Alternatively, the training input data can comprise at least one set of high-resolution 3D T1w GRE MRI data while the training output data comprise at least one set of low-resolution synthetic T1-weighted image data. In this case, the processor module 110, in particular the image transformation module, can provide high-resolution synthetic T1-weighted image data which correspond and match to the training output data with lower spacing/resolution information, thereby avoiding a substantial loss of information due to reduced spacing/resolution.

Moreover, the adaption of the processor module by applying the set of training data 240, preferably of the image transformation module and particularly of such free transfer parameters, can also comprise and refer to, either subsequently (as illustrated in Fig. 3) or as an integral part of the process 240, the training for/of determination and/or assignment 250 of at least one image property value for/to at least one image property parameter, like e.g. an image contrast and/or and image intensity.

The processor module, preferably the image transformation, can be taught and learns a relation between e.g. the image contrast of (training) input data like MRI patient scan data (3D T1w GRE) and the image contrast of the (training) output data to be generated, like synthetic T1-weighted image data, or to be replaced by synthetic image data, e.g. image data from a clinical 2D T1w TSE.

On this basis, the processor module 110 can be trained and configured to generate Bone MRI data as well as synthetic T1-weighted image data on basis of a transfer 246; 250 of the training input data into the training output data, preferably by maintaining as much image information as possible for the synthetic T1-weighted image data to be generated as possible.

Finally, the appropriately configured image transformation module is provided 270.

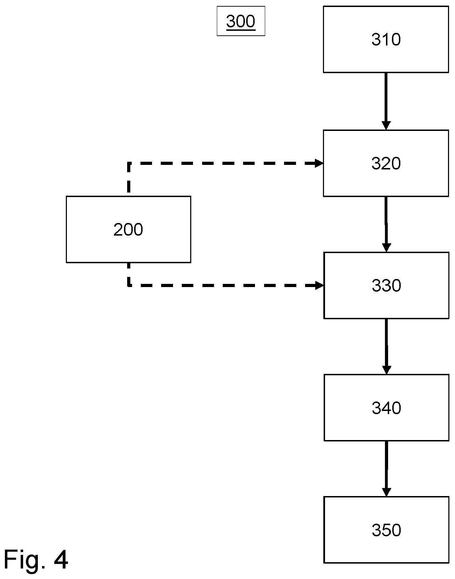

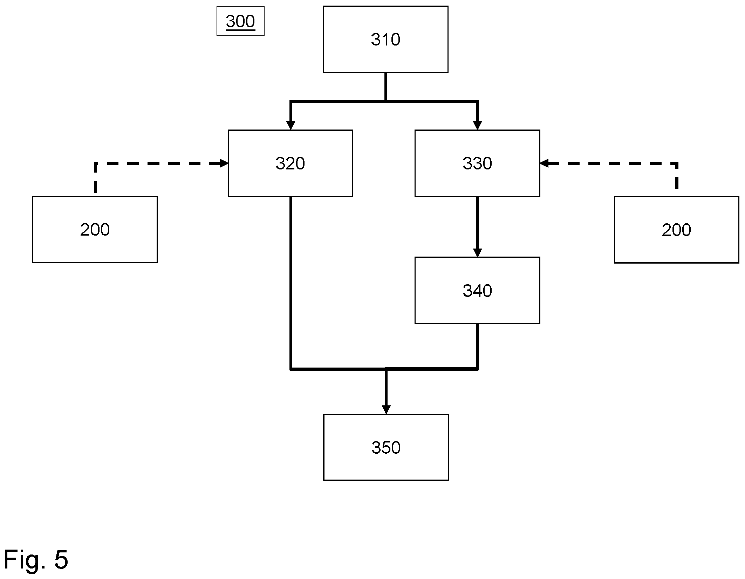

In **Fig. 4** a schematic flow-chart illustrates an embodiment of a method for providing and executing primary and/or secondary data assessment. **Fig. 5** shows a schematic flow-chart for another embodiment of a method for providing and executing primary and/or secondary data assessment.

While Fig. 4 illustrates the subsequent execution of primary data assessment 320 and secondary data assessment 330, Fig. 5 shows an approach for parallel, and preferably simultaneous, primary/secondary data assessment 320; 330.

In more detail, in a first step at least one set of MRI patient scan data are received 310. Moreover, it is possible to receive multiple sets of MRI patient scan data of the first patient and or different sets of imaging data of the first patient, like MRI patient scan data, CT patient scan data, Bone MRI storage data, in particular synthetic CT storage data, synthetic T1-weighted image storage data and/or segmentation data of the first patient.

Multiple different types of image data can be combined as well as also storage data, namely previously gathered data of the first patient being stored on either a storage medium, a (remote) server or the like, can be combined for the primary/secondary data assessment 320; 330.

In a next step, primary data assessment 320 is executed by the processor module 110, namely the Bone MRI module.

Subsequent (see Fig. 4) or in parallel (see Fig. 5) thereto, the secondary data assessment 330 can be executed by the processor module 110, in particular the image transformation module.

In a further step, the processor module 110, in particular the image transformation module can select and image property parameter, in particular an image contrast, image intensity and/or the like, and assign an appropriate image property value 340 to it in order to provide synthetic T1-weigheted image data. Such process 340 can either be executed subsequently/separately (as illustrated in Fig. 5) or as an integral part of e.g. the secondary data assessment 330.

Preferably, the Bone MRI data, e.g. synthetic CT data, and the sT1w/synthetic T1-weighted image data can comprise different T1-weight factors/values, in particular to be able to provide deviating tissue information.

Finally, the Bone MRI data and the sT1w/synthetic T1-weighted image data as provided can be illustrated and/or overlayed 350 in a human perceptible format, namely on a display device like a two- or three-dimensional display means, e.g. a monitor, a threedimension hologram visualization, in form of a augmented/virtual reality illustration or the like.

Moreover, the processor module's 110 Bone MRI module and image transformation module can be trained and configured on basis of training method 200, as illustrated in Fig. 4 and 5 by dotted lines.

In summary, the present invention particularly discloses a system as well as a method for training in order to allow for an improved assessment of different kinds of bodily tissues and/or bodily fluids and/or air, in particular air being associated with a human body, by providing synthetic image data of different kinds/types for a first patient in order to allow for a reduction of separate medical imaging sequences, whereby the loss of information by the generation of such synthetic image data for the first patient is minimized.

Moreover, by providing also a combined training of multiple modules of the processor module, namely the Bone MRI module and the image transformation module, the quality, stability and reliability of the resulting process to generate such synthetic image data on basis of at least one set of MRI patient scan data can be further improved.

Due to such combined training it is even possible that Bone MRI data, namely synthetic CT data and sT1w/synthetic T1-weighted image data, can result in such synthetic CT data providing even better soft tissue contrast, which may allow soft-tissue assessment on Bone MRI data alone, using a dedicated soft-tissue viewing mode.

Furthermore, by providing multiple sets of different image data for a first patient, in particular Bone MRI data and sT1w/synthetic T1-weighted image data, in the same geometry, i.e. position, orientation and resolution, the reading and interpretation of the combined set of images is facilitated for a corresponding (human) user of the system. For example, a combined fusion and visualization of bone and soft tissue, in particular by also implementing segmentation data, may provide a highly improved basis for data assessment by a user, preferably starting from a minimum imaging sequence like a single set of MRI patient scan data of a first patient.

### Reference numerals

- 100: System
- 110: Processor module
- 120: Storage module
- 130: MRI scanning device
- 140: Data connection
- 150: Remote data storage/Server
- 200: Method for training
- 210: Requesting and/or receiving at least one set of training data
- 220: Applying set of training data on processor module
- 230: Applying set of training data on Bone MRI module
- 236: Transfer of training input data to training output data
- 240: Applying set of training data on image transformation module
- 242: Selecting body region data
- 244: Selecting at least one sub-volume data
- 246: Transfer of training input data to training output data
- 250: Assign/determine image property value for image property parameter
- 260: Provide configured Bone MRI module
- 270: Provide configured image transformation module
- 300: Method for primary/secondary data assessment
- 310: Receiving MRI patient scan data of first patient
- 320: Primary data assessment
- 330: Secondary data assessment
- 340: Select image property parameter and assign value
- 350: provide/display human perceptible illustration

## Claims

1. System for assessment of different kinds of bodily tissues and/or bodily fluids and/or air, in particular air being associated with a human body, comprising:
- at least one processor module (110) for handling and processing of MRI patient scan data,
- at least one storage module (120) for storing patient data, in particular MRI patient scan data and/or synthetic CT data and/or data associated therewith, of at least one first patient,
wherein at least one data connection (140) is provided between the processor module (110) and the at least one storage module (120), in particular a bidirectional data connection (140), such that data are transferrable for processing and/or for storing,
wherein the processor module (110) is configured to receive and/or request (310) the at least one set of MRI patient scan data from the storage module (120) or from a MRI scanning device (130),
wherein, for primary data assessment (320), the processor module (110), preferably comprising a Bone MRI module of the processor module (110), is configured to process and/or analyse at least the MRI patient scan data of the first patient such that at least one set of Bone MRI data, in particular synthetic CT data, of the first patient is provided, and/or
wherein, for a secondary data assessment (330), the processor module (110), preferably comprising an image transformation module, is configured to process and/or analyse at least the MRI patient scan data of the first patient such that at least one set of synthetic T1-weighted image data of the first patient is provided.

2. System (100) according to claim 1,
**characterized in that**
the processor module (110), preferably the image transformation module, is configured to select at least one image property parameter of the synthetic T1-weighted image data, in particular an image contrast, image intensity and/or the like, to which at least one image property value is assignable (340) to and/or determinable for at least one medium, in particular a bodily tissue and/or a bodily fluid and/or air, preferably air being associated with the first patient, by the processor module (110), in particular by the image transformation module.

3. System (100) according to one of the preceding claims,
**characterized in that**
for the primary data assessment (320) the processor module (110), in particular the Bone MRI module, is configured to apply a first transfer element on at least the MRI patient scan data, CT patient scan data, Bone MRI storage data, in particular synthetic CT storage data, synthetic T1-weighted image storage data and/or segmentation data of the first patient to provide at least one set of Bone MRI data, in particular at least one set of synthetic CT data, and/or
wherein for the secondary data assessment (330) the processor module (110), in particular the image transformation module, is configured to apply a fourth transfer element on at least the MRI patient scan data, CT patient scan data, Bone MRI storage data, in particular synthetic CT storage data, synthetic T1-weighted image storage data and/or segmentation data of the first patient to provide at least one set of synthetic T1-weighted image data,
wherein the Bone MRI data and the synthetic T1-weighted image data comprise different T1-weight factors.

4. System (100) according to one of the preceding claims,
**characterized in that**
the processor module (110), in particular the Bone MRI module, for primary data assessment (320) is further configured:
- to apply a second transfer element on the at least one set of Bone MRI data, in particular synthetic CT data, and/or MRI patient scan data of the first patient such that reduced patient data of the first patient are generated which provide tissue volume information, in particular water and/or fat volume related information and/or the like, of the first patient, and/or receiving tissue volume information, in particular water and/or fat volume related information and/or the like, from the MRI scanning device (130),
- to apply a third transfer element on the reduced patient data and/or the MRI patient scan data of the first patient such that total bone volume information are achieved,
wherein the processor module (110), in particular the Bone MRI module, is further configured to process and/or analyse the MRI patient scan data, the at least one set of synthetic CT data and/or the reduced patient data, preferably in combination with each other, such that at least one bone volume parameter, in particular bone mineral density, fat percentage, calcium concentration, total density trabecular space and/or the like, of the first patient is determined.

5. System (100) according to one of the preceding claims,
**characterized in that**
the processor module (110), preferably with the Bone MRI module and the image transformation module, is configured to execute primary and secondary data assessment (320; 330) simultaneously or subsequent to each other.

6. System (100) according to one of the preceding claims, in particular according to claim 3,
**characterized in that**
the system further comprises a visualization device, in particular a two- or three-dimensional display means, being configured to provide a human perceptible illustration (350) for a first user of the system of
- at least one set of Bone MRI data, in particular synthetic CT data, of the first patient as provided by the processor module (110), in particular by the Bone MRI module, and/or
- at least one set of synthetic T1-weighted image data as provided by the processor module (110), in particular by the image transformation module, preferably comprising at least one image property value being assigned to and/or determined (340) for at least one image property parameter for at least one medium, in particular a bodily tissue and/or a bodily fluid and/or air, in particular air being associated to a human body.

7. Method (200) for training of a processor module (110), preferably of a Bone MRI module and/or an image transformation module, in particular of a system (100) according to one of the preceding claims,
**characterized in that**
the method (200) comprises the steps of:
- requesting and/or receiving (210) MRI patient scan data, CT patient scan data, Bone MRI data, in particular synthetic CT data, synthetic T1-weighted image data and/or segmentation data of a plurality of patients as at least one set of training data comprising training input data and training output data,
- applying (220; 230; 240) the at least one set of training data to the processor module (110), preferably to the Bone MRI module and/or the image transformation module, for training of the Bone MRI module, in particular the first transfer element, and/or of the image transformation module, in particular the fourth transfer element, to be configured to generate
∘ at least one set of Bone MRI data (238), in particular synthetic CT data, by the processor module (110), preferably by the Bone MRI module,
and/or
∘ at least one set of synthetic T1-weighted data (248) by the processor module (110), preferably by the image transformation module,
wherein training of the processor module (110) is preferably achieved by optimizing at least one free transfer parameter of the Bone MRI module, in particular the first transfer element, and/or at least one free transfer parameter of the image transformation module, in particular of the fourth transfer element, and wherein training output data are preferably Bone MRI data and/or synthetic T1-weighted image data of the plurality of patients.

8. Method (200) according to claim 7,
**characterized in that**
on basis of the at least one set of training data of the plurality of patients, the processor module (110), preferably the image transformation module of the processor module (110), in particular the fourth transfer element, is trained to be configured to assign and/or determine at least one image property value (250) to at least one image property parameter, in particular an image contrast, image intensity and/or the like, for at least one medium, in particular a bodily tissue and/or a bodily fluid and/or air, of the synthetic T1-weighted image data, and/or wherein the processor module (110), preferably the image transformation module, in particular the fourth transfer element, comprises a machine learning element, a deep learning element, a neural network element and/or the like.

9. Method (200) according to claim 7 or 8,
**characterized in that**
applying (220; 230; 240) the at least one set of training data for training of the image transformation module, in particular of the fourth transfer element, further comprises the following steps:
- selecting (242) body region data, in particular at least one two- or three-dimensional volume, from the at least one set of training data, in particular from the training input data, with the body region data comprising and/or being separated into a set of multiple sub-volume data, preferably a stack of multiple sub-volume data, in particular multiple two- or three-dimensional sub-volumes,
- applying the selected body region data, in particular the multiple sub-volume data, to the processor module (110) such that the processor module (110), preferably the image transformation module, in particular the fourth transfer element, transfers (246) the corresponding training input data to the corresponding training output data in order to be configured to generate (248) at least one set of synthetic T1-weighted image data.

10. Method (200) according to claim 9,
**characterized in that**
applying (240) the at least one set of training data, in particular the selected body region data, for training of the image transformation module further comprises the following steps:
- selecting (244) at least one sub-volume data from the training input data and at least one, preferably one, corresponding sub-volume data from the training output data,
- providing and applying at least one resampling operation parameter as the at least one free transfer parameter of the fourth transfer element to the at least one selected sub-volume data in order to modify the geometry of the selected sub-volume data, in particular to modify at least one position parameter, an orientation parameter, a spacing parameter or the like of the selected sub-volume data, by assigning a resampling operation value to the resampling operation parameter
such that the processor module (110), in particular the image transformation module, is configured to transfer (246) a geometry of training input data, in particular of the selected sub-volume data, to the corresponding training output data, in particular to the corresponding sub-volume data of the training output data.

11. Method (200) according to one of claims 9 to 11,
**characterized in that**
applying (240) the at least one set of training data, in particular the selected body region data, for training of the image transformation module further comprises the following steps:
- selecting (244) at least one sub-volume data from the training input data and at least one, preferably one, corresponding sub-volume data from the training output data,
- extracting geometry meta-data from the training input data and applying (248) such geometry meta-data to the processor module (110), in particular to the image transformation module, such that the processor module (110), in particular the image transformation module, is configured to modify at least one geometry parameter, in particular at least one position parameter, an orientation parameter, a spacing parameter or the like of the selected sub-volume data, as the at least one free transfer parameter of the fourth transfer element
in order to transfer (246) a geometry of training input data, in particular of the selected sub-volume data, to the corresponding training output data, in particular to the corresponding sub-volume data of the training output data.

12. Method (200) according to one of claims 9 to 12,
**characterized in that**
applying (240) the at least one set of training data, in particular the selected body region data, for training of the image transformation module further comprises the following steps:
- selecting (244) multiple sub-volume data of the training input data and at least one, preferably one, corresponding sub-volume data from the training output data,
- providing and applying at least one resampling operation parameter as the at least one free transfer parameter of the fourth transfer element to the at least one selected sub-volume data in order to modify the geometry of the selected sub-volume data, in particular to modify at least one position parameter, an orientation parameter, a spacing parameter or the like of the selected sub-volume data, by assigning at least one resampling operation value to the resampling operation parameter
such that the processor module (110), in particular the image transformation module, is configured to transfer (246) a geometry of training input data, in particular of the selected sub-volume data, to the corresponding training output data, in particular to the corresponding sub-volume data of the training output data,
wherein the at least one resampling operation value is randomized, preferably on basis of a uniform random distribution or the like,
wherein within the selected body region data different resampling operation values are applicable for and assigned to at least two of the multiple sub-volume data of the training input data,
wherein during subsequent executions of the method different selections, in particular shifted selections within the selected body region data, of the multiple sub-volume data from the training input data are used and the selection of sub-volume data from the training output data maintains the same.

13. Method (200) according to one of claims 10 to 12,
**characterized in that**
the step of providing and applying at least one resampling operation parameter as free transfer parameter of the fourth transfer element further comprises the following steps:
- identifying a two- or three-dimensional profile of the at least one sub-volume data of the training input data,
- pre-determining and/or optimizing at least one resampling operation value on basis of the identified profile for the respective sub-volume data,
- applying the at least one pre-determined and/or optimized resampling operation value to at least one of the multiple sub-volume data of the training input data,
such that the processor module (110), in particular the image transformation module, is configured to transfer (246) a geometry of training input data, in particular of the selected sub-volume data, to the corresponding training output data, in particular to the corresponding sub-volume data of the training output data.

14. Method for providing a primary data assessment (320) and/or a secondary data assessment (330) by a system (100) according to one of claims 1 to 6.

15. Computer-readable medium comprising instructions which cause at least one computer, at least one processor and/or the like to execute the method (200) for training of the processor module (110), in particular of the Bone MRI module and/or the image transformation module, according to claims 7 to 13 and/or to execute the method (300) according to claim 14.
